# EUROPEAN PATENT APPLICATION

(11) **EP 4 079 337 A1**
(43) Date of publication of application: **26.10.2022**
(21) Application number: 22168666.0
(22) Date of filing: 15.04.2022
(51) Int. Cl.: A61L 2/28, A61L 2/07, C12Q 1/22

(54) **AUTOCLAVE STERILISATION SYSTEM AND CORRESPONDING METHOD TO CARRY OUT A STERILISATION CYCLE**

(30) Priority: 19.04.2021 IT 202100009833
(71) Applicant: Archa S.r.l., 56121 Pisa (IT); S.M.I. Medical S.r.l., 54033 Carrara (MS) (IT)
(72) Inventor: GAMBINERI, Francesca, 56127 Pisa (IT); CERVELLI, Fabrizio, 55049 VIAREGGIO (LU) (IT); BAZZICHI, Agostino, 56019 VECCHIANO (PI) (IT); DE PIAN, Eros, 00119 ROMA (IT)
(74) Representative: Studio Torta S.p.A.

(57) **Abstract**

An autoclave sterilization system (1) having: a container (2), which encloses a sterilization chamber (3) and has at least one wall (6) delimiting the sterilization chamber (3) and a through opening (13) directly communicating with the sterilization chamber (3); a vacuum generating device (7), which is configured to create a vacuum inside the sterilization chamber (3); a feeding device (8) to feed saturated steam into the sterilization chamber (3); at least one tool (9, 10) to check the sterilization process; at least one checking chamber (11, 12), which is arranged on the outside of the sterilization chamber (3) and is configured to at least partially house the checking tool (9, 10); and a vertical duct (14, 15), which is connected to the through opening (13) and ends, at the opposite end relative to the through opening (13), in the checking chamber (11, 12) so as to lead, during a sterilization treatment, the saturated steam present in the sterilization chamber (3) into the checking chamber (11, 12).

## Description

### CROSS-REFERENCE TO RELATED APPLICATIONS

This Patent Application claims priority from Italian Patent Application No. 102021000009833 filed on 19 April 2021.

### TECHNICAL FIELD

The present invention relates to an autoclave sterilization system and to a corresponding method to carry out a sterilization cycle.

### PRIOR ART

The sterilization of medical devices is one of the main moments in the process for preventing and controlling infections and, among the most used sterilization methods, there is the use of saturated steam in an autoclave since it is a process which is inexpensive, non-toxic, and has a good penetration ability.

An autoclave sterilization system comprises a sterilization chamber inside which a vacuum is initially created and into which pressurized saturated steam is subsequently fed (for example at the temperature of 134°C and at the pressure of 2.1 bar for an amount of time ranging between 5 and 7 minutes, or at the temperature of 121°C and at the pressure of 1.1 bar for an amount of time ranging between 15 and 20 minutes).

A sterilization cycle can be monitored by means of chemical or biological indicators for checking the achievement of the desired outcome, i.e. the reaching of sterility inside the sterilization chamber at the end of the sterilization cycle. In particular, at the beginning of the sterilization cycle, (at least) a sterilization indicator is inserted into the sterilization chamber, said sterilization indicator being sensitive to all three parameters necessary for the sterilization (temperature, pressure, time): at the end of the sterilization cycle, the sterilization indicator is analysed so as to check that it was subjected to a suitable temperature and to a suitable pressure for a suitable amount of time.

Much use is made (as provided for by various regulations) of biological sterilization indicators which are normally composed of vials containing spores, typically *"Geobacillus Stearothermophilus"* spores (this bacterium is commonly used as sample organism for the sterilization validation studies and for the periodical checks of the sterilization cycles). Once the sterilization cycle ends, the (sealed) vial which was subjected to the sterilization cycle is broken for inserting the spores contained therein into a culture medium and thus proceeding with the incubation of the spores; a colour change or a turbidity change indicates the outcome of the sterilization process: if there are no changes, the sterility conditions have been met, otherwise the growth of the spores indicates that the sterilization process did not take place correctly.

Patent No. US4591566A1 describes a system for monitoring the sterilization of biologically contaminated medical material carried out in autoclave by means of the insertion into a central zone of the autoclave of a biological sterilization indicator represented by a sealed vial containing *"Geobacillus Stearothermophilus"* spores; in particular, a test probe is used provided with a rod made of heat-resistant material having an end chamber closable by a rotatable sleeve and is adapted to house the vial (the rotation of the sleeve locks the vial in place). A portion of the handle of the test probe can have a recess which extends laterally and contains a thermocouple with wires extending through the rod by means of a connector for the connection to an external thermometer for directly monitoring the temperature of the material to be sterilized while the test vial is exposed to the high-temperature steam.

However, the confidence in the check of the sterilization cycle can be (negatively) influenced by the position in which the test vial is arranged inside the autoclave: in other words, if the test vial is placed in central position inside the autoclave, then the vial is subjected during the sterilization cycle to temperatures that are probably higher than objects being sterilized arranged on the edges of the autoclave and thus it is not a reliable representation of the *"worst case".*

Furthermore, the management of the test vial for checking the sterilization cycle is rather long and laborious, requiring a series of operations that must be manually performed by an operator; as a consequence, the management of the test vial for checking the sterilization cycle is strongly influenced by subjective factors that make the checking of the sterilization cycle less objective than what is desirable.

Finally, it is possible to start analysing the test vial only when the sterilization cycle is totally concluded (thus also at the end of the final cooling and drying step) and it is possible to access the sterilization chamber for removing the test vial; therefore, the outcome of the analysis of the test vial is available only much time after (from a minimum of several hours after up to even several days after) the conclusion of the sterilization cycle.

Patent application No. EP1704873A1 is the most recent prior art and describes a method for characterizing the bioindicators in the sterilization processes by means of autoclave and the like, wherein a bioindicator is kept in the same conditions as the sterilization chamber and extracted after a predetermined time interval; for such purpose, the sterilization chamber is provided with a tube (arranged on the inside or on the outside of the sterilization chamber) to whose ends valves are mounted which allow the insertion from above and the following extraction from below of a bioindicator sample.

### DESCRIPTION OF THE INVENTION

The object of the present invention is to provide an autoclave sterilization system which allows solving the above-described drawbacks, i.e. allows making the management of the biological sterilization indicators for checking the sterilization cycle simpler, faster, and less influenced by subjective factors.

According to the present invention, an autoclave sterilization system and a corresponding method for carrying out a sterilization cycle are provided, according to what is claimed in the appended claims.

The claims describe preferred embodiments of the present invention forming integral part of the present description.

### BRIEF DESCRIPTION OF THE DRAWINGS

The present invention will now be described with reference to the accompanying drawings, which illustrate a non-limiting example embodiment thereof, wherein:
- Figure 1 is a schematic and partial view of an autoclave sterilization system manufactured in accordance with the present invention;
- Figure 2 is a view, on an enlarged scale, of a detail of Figure 1;
- Figure 3 is a perspective and partially exploded view of some parts of the autoclave sterilization system of Figure 1;
- Figure 4 is a perspective and partially sectional view of a checking tool of the autoclave sterilization system of Figure 1;
- Figure 5 is a perspective and partially sectional view of a further checking tool of the autoclave sterilization system of Figure 1; and
- Figure 6 is a longitudinal sectional view of the checking tool of Figure 5.

### PREFERRED EMBODIMENTS OF THE INVENTION

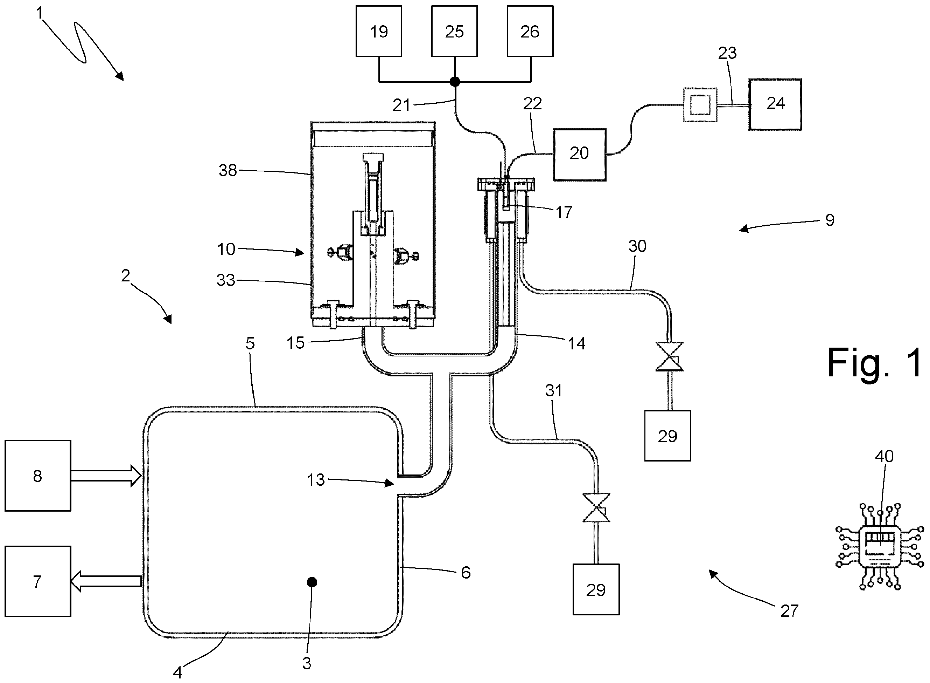

In Figure 1, reference numeral 1 indicates, as a whole, an autoclave sterilization system 1.

The sterilization system 1 comprises a container 2 which encloses a sterilization chamber 3 and obviously has (at least) a door which can be opened for allowing the access to the sterilization chamber 3 (i.e. for inserting the objects to be sterilized into the sterilization chamber 3 and for subsequently removing the sterilized objects from the sterilization chamber 3).

In the embodiment illustrated in the accompanying figures, the container 2 has a parallelepiped shape (but it could also have a different shape) and comprises a lower wall 4 which constitutes a bottom of the sterilization chamber 3 (i.e. which delimits the sterilization chamber 3 at the bottom), an upper wall 5 which constitutes a ceiling of the sterilization chamber 3 (i.e. which delimits the sterilization chamber 3 at the top), and a lateral wall 6 which constitutes the sides of the sterilization chamber 3 (i.e. which delimits the sterilization chamber 3 on the sides) and which connects the lower wall 4 and the upper wall 5 to one another.

The sterilization system 1 comprises a vacuum generating device 7 which is connected to the sterilization chamber 3 and is configured to create a vacuum inside the sterilization chamber 3. Furthermore, the sterilization system 1 comprises a feeding device 8 which is connected to the sterilization chamber 3 and is configured to feed pressurized saturated steam into the sterilization chamber 3.

The sterilization system 1 comprises two different checking tools 9 and 10 for checking the sterilization process; i.e. the two checking tools 9 and 10 are adapted to determine, by obviously using different and complementary checking methods, whether or not the sterilization process inside the sterilization chamber 3 took place correctly.

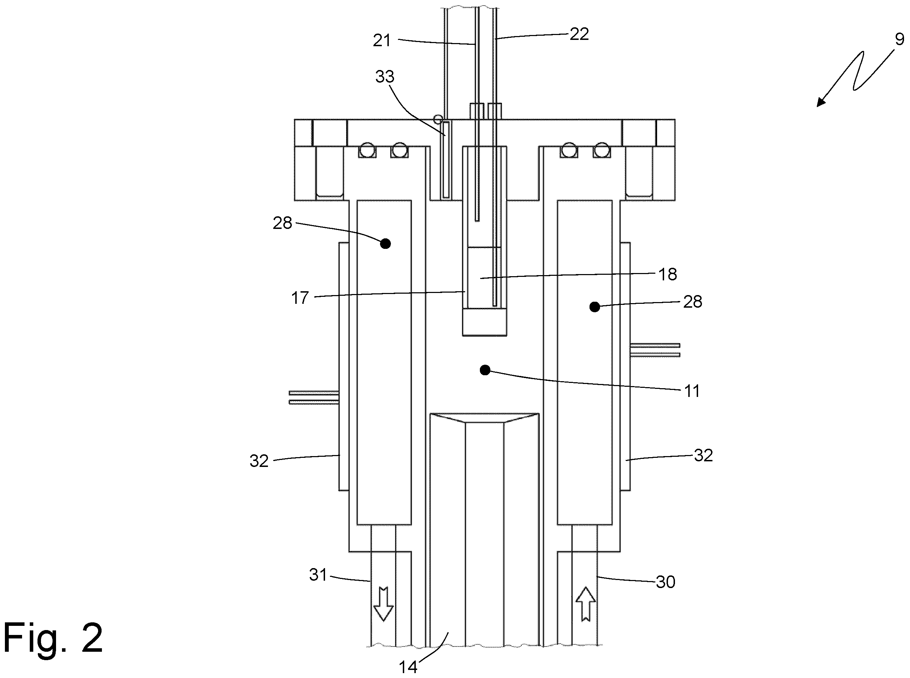

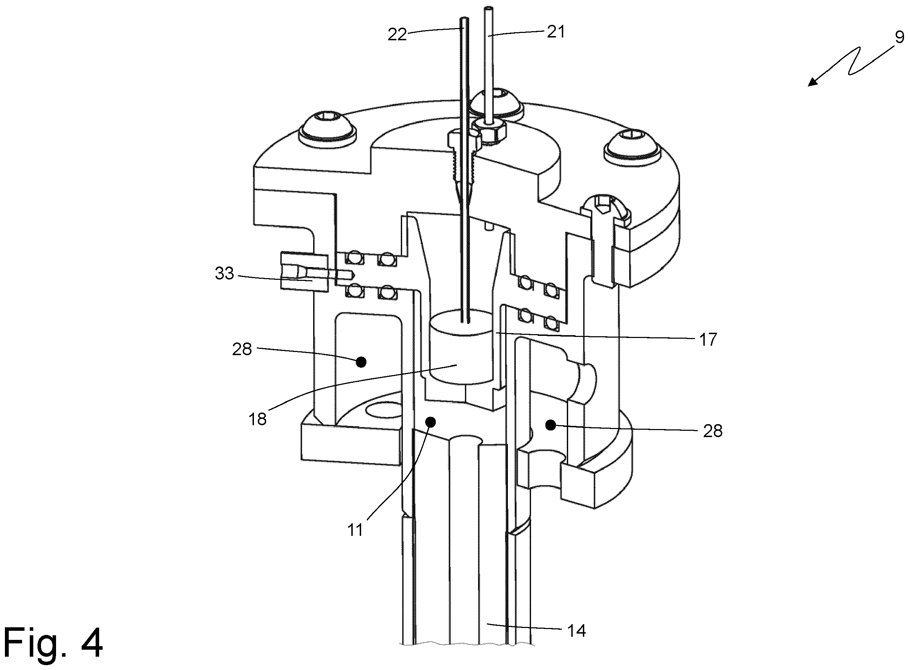

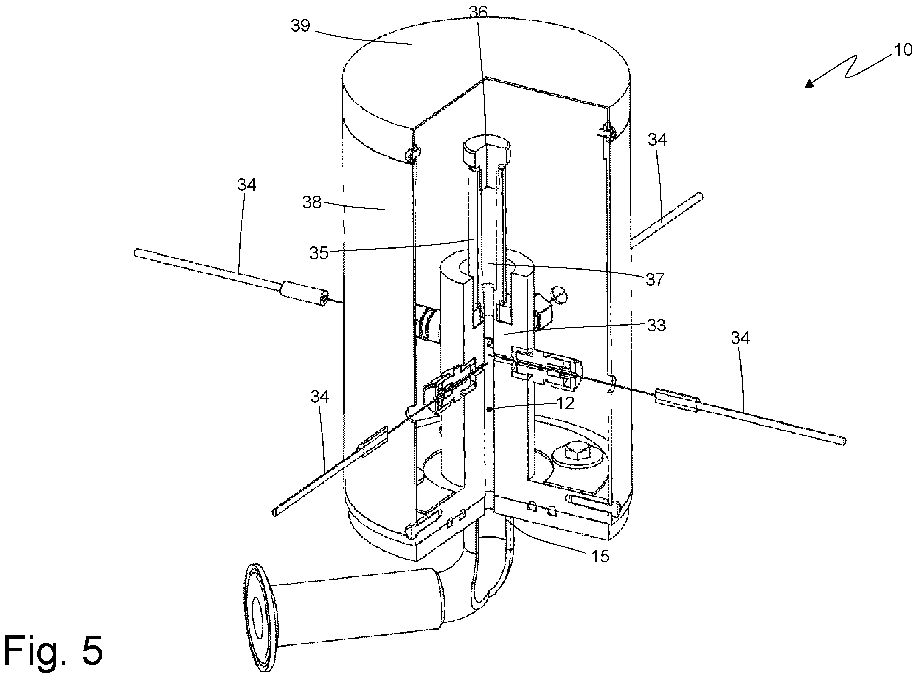

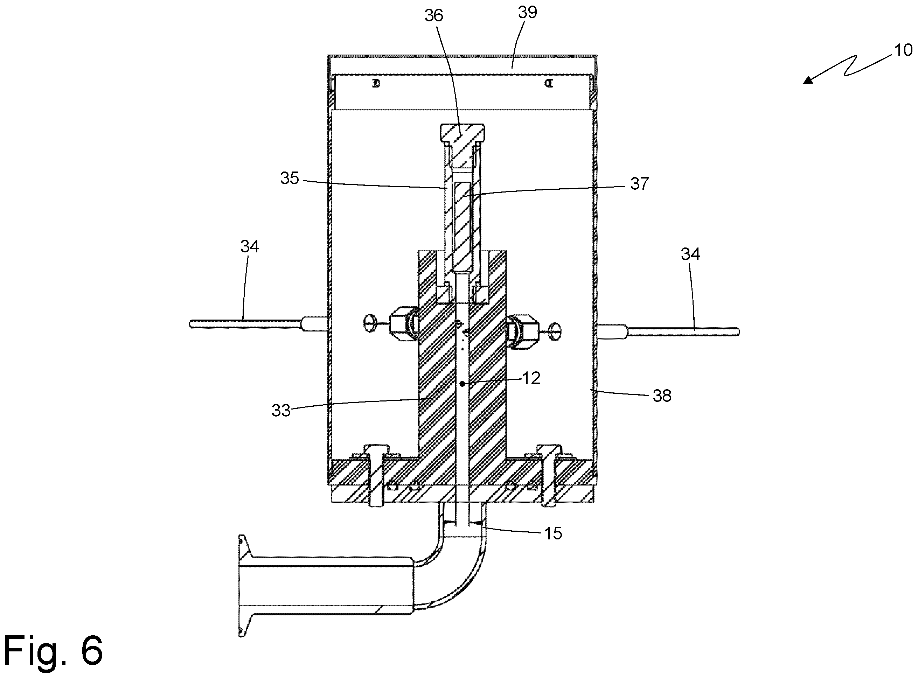

The sterilization system 1 comprises two different and separate checking chambers 11 and 12 (better illustrated in Figures 2 and 4 with regard to the checking chamber 11 and in Figures 5 and 6 with regard to the checking chamber 12), which are configured to (at least partially) house the checking tools 9 and 10; i.e. at least the sensitive parts of the checking tools 9 and 10 are arranged inside respective checking chambers 11 and 12. In particular, the checking chamber 11 at least partially houses the checking tool 9 and the checking chamber 12 at least partially houses the checking tool 10.

The lateral wall 6 of the container 2 comprises a through opening 13 directly communicating with the sterilization chamber 3. The through opening 13 is arranged between a ceiling (defined by the upper wall 5) of the sterilization chamber 3 and a bottom (defined by the lower wall 4) of the sterilization chamber 3; preferably, the through opening 13 is arranged at a height ranging between 60% and 70% of a distance between the ceiling and the bottom of the sterilization chamber 3 (i.e. the through opening 13 is arranged at approximately 2/3 of the height of the sterilization chamber 3).

According to other embodiments not illustrated, the through opening 13 could be arranged in a different position of the lateral wall 6 of the container 2 (i.e. at a greater height and thus closer to the upper wall 5 or at a lower height and thus closer to the lower wall 4. Alternatively, the through opening 13 is not obtained through the lateral wall 6 of the container 2 but is obtained through the upper wall 5 of the container 2.

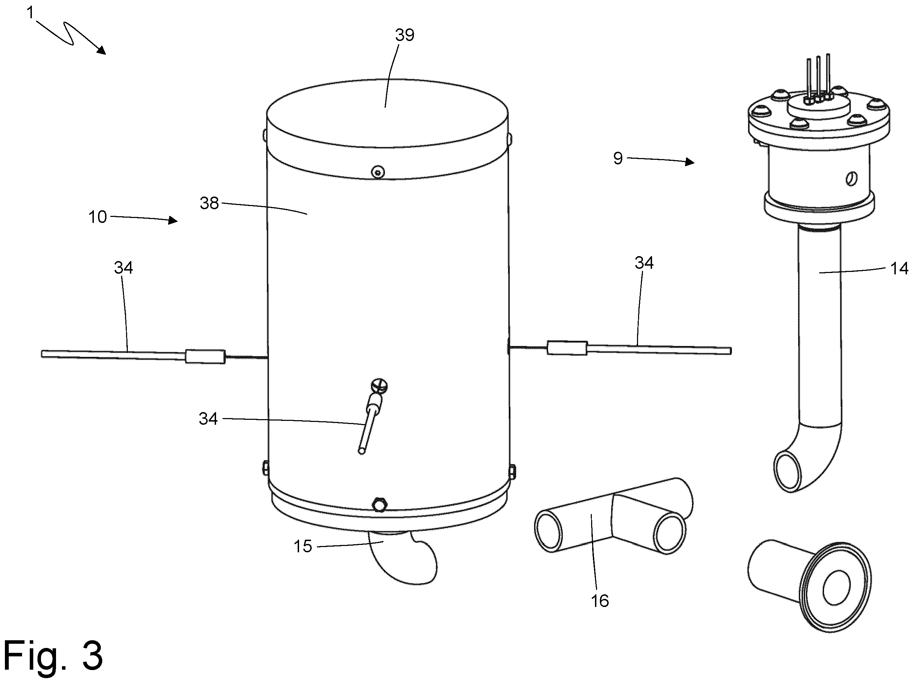

The sterilization system 1 comprises two different and separate vertical ducts 14 and 15, each of which is connected to the through opening 13 and ends, at the opposite end relative to the through opening 13, at a corresponding checking chamber 11 or 12 so as to lead, during a sterilization treatment, the vacuum (first) and the saturated steam (subsequently) present in the sterilization chamber 3 into the corresponding checking chamber 11 or 12. In particular, the vertical duct 14 connects the checking chamber 11 to the through opening 13, whereas the vertical duct 15 connects the checking chamber 12 to the through opening 13. According to what is illustrated in Figure 3, both vertical ducts 14 and 15 (which are adjacent and parallel to one another) originate from the same and single through opening 13 (which is thus shared with the two vertical ducts 14 and 15) by means of a forked duct 16 preferably arranged horizontally.

Preferably, both checking chambers 11 and 12 are arranged at a vertical height that is greater than a vertical height of the ceiling (defined by the upper wall 5) of the sterilization chamber 3; i.e. both checking chambers 11 and 12 are arranged at a greater height than the ceiling of the sterilization chamber 3. According to other embodiments not illustrated, both checking chambers 11 and 12 (or also one of the two checking chambers 11 and 12) are arranged at a vertical height that is less than a vertical height of the ceiling (defined by the upper wall 5) of the sterilization chamber 3.

The checking chambers 11 and 12 (which communicate with the sterilization chamber 3) are upper appendixes of the sterilization chamber 3 in which possible and undesired air bubbles (not correctly evacuated by the vacuum generating device 7) are more likely to remain and in which the pressurized saturated steam fed by the feeding device 8 tends to arrive with greater difficulty. As a consequence, the checking chambers 11 and 12 are the most *"critical"* zone of the entire sterilization chamber 3, i.e. the most difficult zone in which to create a vacuum and, at the same time, the most difficult zone to heat with the pressurized saturated steam; therefore, if the sterilization treatment (i.e. the application of heat and pressure for a sufficient amount of time) takes place correctly in the checking chambers 11 and 12, then it certainly takes place correctly also in all of the sterilization chamber 3 and if the sterilization treatment (i.e. the application of heat and pressure for a sufficient amount of time) should not take place correctly, then the greater problems thereof are certainly manifested in the checking chambers 11 and 12.

Various experimental tests have highlighted that the most unfavourable sterilization conditions (i.e. with lower temperature) occur always and only in the checking chambers 11 and 12; these experimental tests show beyond a reasonable doubt that the checking chambers 11 and 12 are certainly *"the worst case"* and that therefore if a suitable thermal treatment is anyway carried out in the checking chambers 11 and 12, then a suitable thermal treatment was certainly carried out also in all of the sterilization chamber 3.

According to what is illustrated in Figures 2 and 4, the checking tool 9 comprises a test receptacle 17 which is configured to contain a liquid-state biological sterilization indicator 18, is arranged in the checking chamber 11, and is pneumatically and hydraulically isolated relative to the checking chamber 11.

As mentioned in the foregoing, the checking chamber 11 directly communicates with the vertical duct 14 so that, during a sterilization treatment, the checking chamber 11 is filled with the saturated steam present in the sterilization chamber 3, which brushes (and thus heats) the test receptacle 17 from the outside.

Preferably, the liquid-state biological sterilization indicator 18 contains *"Geobacillus Stearothermophilus"* spores (this bacterium is commonly used as sample organism for the sterilization validation studies and for the periodical checks of the sterilization cycles).

The test receptacle 17 is reusable and permanent, i.e. the checking chamber 11 is configured to house the test receptacle 17 in a permanent (non-removable) manner. According to a preferred but not binding embodiment, the test receptacle 17 is made of metallic material (which is an excellent heat conductor) and in particular of aluminium (in order to have a reduced mass and thus also a reduced thermal inertia); in this manner, the saturated steam present in the checking chamber 11 can quickly heat the liquid-state biological sterilization indicator 18 contained in the test receptacle 17.

According to what is illustrated in Figure 1, the checking tool 9 comprises a feeding device 19, which is configured to feed, before carrying out a sterilization treatment, a quantity of liquid-state biological sterilization indicator 18 into the test receptacle 17; the feeding device 19 comprises a tank (possibly refrigerated in order to prevent undesired germinations) containing the liquid-state biological sterilization indicator 18 and a pump that sucks a quantity of the liquid-state biological sterilization indicator 18 out of the tank for feeding it into the test receptacle 17.

Furthermore, the checking tool 9 comprises a removing device 20 configured to suck, after carrying out a sterilization treatment, the quantity of liquid-state biological sterilization indicator 18 out of the test receptacle 17. The removing device 20 comprises a pump which is capable of sucking the quantity of liquid-state biological sterilization indicator 18 present in the test receptacle 17 as a consequence emptying the test receptacle 17.

In particular, an inlet pipe 21 is provided which originates from the feeding device 19 and ends inside the test receptacle 17, and an outlet pipe 22, different and separate from the inlet pipe 21, is provided which originates from the test receptacle 17 (in particular from the bottom of the test receptacle 17) and ends in the removing device 20.

The checking tool 9 comprises an analysis device 23 which is configured to receive the quantity of liquid-state biological sterilization indicator 18 from the removing device 20 and thus to analyse the liquid-state biological sterilization indicator 18 so as to check whether a sterilization treatment had a positive result; in particular, the analysis device 23 researches any presence of germination activity in the quantity of liquid-state biological sterilization indicator 18 so as to establish whether the sterilization cycle took place correctly: in the complete absence of germination activity, then, all the *"Geobacillus Stearothermophilus"* spores were killed by the sterilization cycle and thus the sterilization cycle took place correctly, whereas in the (even minimum) presence of germination activity, not all the *"Geobacillus Stearothermophilus"* spores were killed by the sterilization cycle and thus the sterilization cycle did not take place correctly and has to be repeated.

The liquid-state biological sterilization indicator 18 could contain a substance that changes its colour in the presence of a pH change: the germination activities of the *"Geobacillus Stearothermophilus"* spores determine a pH variation and thus cause a colour change of the liquid-state biological sterilization indicator 18. As a consequence, the analysis device 23 could comprise an optic sensor (for example a spectrophotometer) that detects with extreme precision and sensitivity the colour of the liquid-state biological sterilization indicator 18.

The checking tool 9 finally comprises a collecting device 24 which is arranged downstream of the analysis device 23 and is intended to collect (for the following disposal) the quantity of liquid-state biological sterilization indicator 18 once the analysis in the analysis device 23 has ended (i.e. once the quantity of liquid-state biological sterilization indicator 18 has ended its function).

According to a different embodiment, the checking tool 9 is devoid of the analysis device 23 and the removing device 20 feeds the quantity of liquid-state biological sterilization indicator 18 sucked out of the test receptacle 17 into a vial (or similar transportable container) which is subsequently removed manually and analysed in a known manner outside of the checking tool 9.

The checking tool 9 comprises a feeding device 25 which is configured to feed a washing liquid (typically water) into the test receptacle 17 and a feeding device 26 which is configured to feed a sterilization liquid into the test receptacle 17; generally, the three feeding devices 19, 25 and 26 share the same inlet pipe 21 since the three feeding devices 19, 25 and 26 are always used in different moments.

It is important to observe that it is not necessary to carry out washing and/or sterilization cycles of the test receptacle 17 during the continuous use of the sterilization system 1, since the content of the test receptacle 17 is (hopefully) sterilized at each use; it could instead be advisable (even if not strictly necessary) to carry out a washing and/or sterilization cycle of the test receptacle 17 occasionally and also before a prolonged interruption of the sterilization system 1.

According to what is illustrated in Figure 1, the checking tool 9 comprises a thermoregulation system 27 which is configured to keep the checking chamber 11 containing the test receptacle 17 at a desired incubation temperature, i.e. at a temperature that can maximise the germination of the *"Geobacillus Stearothermophilus"* spores present in the quantity of liquid-state biological sterilization indicator 18; obviously, the thermoregulation system 27 is used only when the thermal treatment of the sterilization cycle is concluded, i.e. when the feeding of saturated steam into the sterilization chamber 3 is concluded.

The thermoregulation system 27 comprises a liquid cooling device provided with a cooling chamber 28 (better illustrated in Figures 2 and 4) which surrounds the checking chamber 11 and is isolated from the checking chamber 11; moreover, the cooling device is provided with a circulation device 29 which is configured to have a cooling liquid (typically water) circulate in the cooling chamber 28. Generally, the circulation device 29 uses common running water, i.e. takes water at room temperature from a tap, said water, after passing through the cooling chamber 28, is discharged into the drainage system; to such purpose, it is important to observe that the checking chamber 11 is very small and therefore the amount of running water necessary for its cooling is definitely modest. In particular, an inlet pipe 30 is provided which leads the cooling liquid into the cooling chamber 28 and an outlet pipe 31 which removes the cooling liquid from the cooling chamber 28.

The thermoregulation system 27 comprises at least one electrical resistance 32 (schematically illustrated in Figure 2) which is configured to heat the checking chamber 11; it is important to observe that initially it is necessary to cool the checking chamber 11 so as to bring the checking chamber 11 from the sterilization temperature (way above 100°C) to the incubation temperature (approximately 57°C for the *"Geobacillus Stearothermophilus")* and subsequently it is necessary to heat the checking chamber 11 so as to keep the checking chamber 11 at the incubation temperature which is substantially above the room temperature. At the incubation temperature, the (maximum) proliferation of the *"Geobacillus Stearothermophilus"* takes place; therefore, the presence of the electrical resistance 32 (i.e. of a specific heating device) is essential for ensuring the constancy of the incubation temperature substantially above the room temperature.

Finally, the thermoregulation system 27 comprises a temperature sensor 34 (illustrated in Figures 2 and 4) which is configured to measure the temperature of the checking chamber 11 and is used for feedback controlling the thermoregulation system 27.

According to a different embodiment not illustrated, the permanent test receptacle 17 is replaced by a disposable and sealed test receptacle which is manually arranged inside the checking chamber 11 at the beginning of the sterilization cycle and is manually removed from the checking chamber 11 at the end of the sterilization cycle; in this embodiment, the feeding devices 19, 25 and 26, the removing device 20 and the analysis device 23 are not of course provided, whereas the thermoregulation system 27 could be anyway provided, said system allowing keeping the checking chamber 11 (containing the disposable test receptacle) at the incubation temperature for a certain amount of time. As a consequence, the checking chamber 11 is configured to house the disposable test receptacle in a removable manner and has an openable lid that can be opened for inserting or removing a disposable test receptacle.

According to what is illustrated in Figures 5 and 6, the checking chamber 12 is defined in a dead pipe 33 which is arranged vertically so as to place at the top a closed end and so as to place at the bottom an open end which directly communicates with the vertical duct 15, so that, during a sterilization treatment, the saturated steam (coming from the sterilization chamber 3 through the through opening 13) fills the checking chamber 12. The checking tool 10 comprises a plurality (four in the embodiment illustrated in the accompanying figures) of temperature sensors 34 which are arranged at different heights of the dead pipe 33 for detecting respective temperatures inside the checking chamber 12 (defined in the dead pipe 33) and in the proximity of the closed end of the dead pipe 33. According to other embodiments not illustrated, a different number of temperature sensors 34 is provided: generally, from a minimum of one single temperature sensor 34 to a maximum of 6-8 temperature sensors 34.

In this manner, a possible air bubble present inside the checking chamber 12 also having relatively small dimensions (if compared to the overall volume of the sterilization chamber 3) involves one or more temperature sensors 34; i.e. if a possible air bubble is present in the checking chamber 12, the sensitive part of one or more temperature sensors 34 is inside the air bubble and thus does not enter into contact with the saturated steam (which remains below the air bubble) and thus these temperature sensors 34 involved by the air bubble detect a temperature greatly below the temperature of the saturated steam. Therefore, by analysing the measurements provided by the temperature sensors 34 it is both possible to check the presence of a possible air bubble inside the checking chamber 12, and estimate the volume of the possible air bubble (the more temperature sensors 34 arranged at different heights feel the air bubble, i.e. are involved by the air bubble, the bigger the air bubble has to be).

Preferably, the checking chamber 12 has, between the temperature sensor 34 arranged at a greater height and its closed end, a reduced volume ranging between 1.5 and 4.5 cm³; in this manner, the checking chamber 12 can be suitably sensitive also to small air bubbles.

Thanks to its conformation, the checking tool 10 is capable of carrying out an equivalent of the Bowie-Dick test (also called Brown test) for controlling the correct operation of the autoclave sterilization system 1; in fact, as it occurs in the Bowie-Dick test, the checking tool 10 allows checking the removal ability of the air and subsequent penetration ability of the saturated steam.

According to a possible embodiment illustrated in the accompanying figures, at the top of the dead pipe 33 a tubular housing 35 is obtained which is provided with a plug 36 and is configured to contain (in a removable manner) a disposable and sealed test receptacle 37 which is manually arranged inside the housing 35 at the beginning of the sterilization cycle and is manually removed from the housing 35 at the end of the sterilization cycle. The housing 35 is generally metallic, preferably made of aluminium, and is in direct contact with the dead pipe 33 (which instead is preferably made of Teflon or other similar materials); in this manner, the heat transmitted by the saturated steam which is in the checking chamber 12 (i.e. which is inside the dead pipe 33) is transmitted by thermal conduction also to the housing 35 and thus heats the disposable test receptacle 37. Obviously, the disposable test receptacle 37 constitutes a redundant *"duplicate"* of the test receptacle 17; therefore, the disposable test receptacle 37 is used in place of the test receptacle 17 or in addition to the test receptacle 17 when wanting to make a further redundant check relative to the check made by means of the test receptacle 17.

Preferably, the dead pipe 33 and the housing 35 are inserted in a cylindrical protection container 38 provided with a removable plug 39 which allows an easy and fast access from above to the inside of the protection container 38.

Finally, the sterilization system 1 comprises a control unit 1 (schematically illustrated in Figure 1) which superintends the operation of the sterilization system 1.

The modes for carrying out a sterilization cycle by means of the sterilization system 1 are described in the following.

Initially, at least one object to be sterilized is inserted into the sterilization chamber 3 and subsequently the sterilization chamber 3 is closed and hermetically sealed. At this point, the control unit 40 feeds a quantity of liquid-state biological sterilization indicator 18 automatically (i.e. without the need for the intervention of an operator) and using the feeding device 19 in the test receptacle 17 of the checking tool 9. Subsequently, the control unit 40 activates the vacuum generating device 7 for creating a vacuum inside the sterilization chamber 3 and, as a consequence, also inside the checking chambers 11 and 12 (which are directly connected to the sterilization chamber 3). Once reached the desired degree of vacuum, the control unit 40 deactivates the vacuum generating device 7 and activates the feeding device 8 for feeding pressurized saturated steam into the sterilization chamber 3 and, as a consequence, also into the checking chambers 11 and 12 (which are directly connected to the sterilization chamber 3); the presence of the pressurized saturated steam is maintained for a predetermined sterilization amount of time (for example at the temperature of 134°C and at the pressure of 2.1 bar the sterilization amount of time ranges between 5 and 7 minutes, or at the temperature of 121°C and at the pressure of 1.1 bar the sterilization amount of time ranges between 15 and 20 minutes).

Obviously, more complex sterilization cycles can be provided in which steps, wherein a vacuum is created, and steps, wherein pressurized saturated steam is fed, alternate several times.

During the sterilization amount of time, i.e. during the thermal treatment, the control unit 40 uses the checking tool 10 for monitoring the progress of the sterilization cycle; in particular, the control unit 40 reads the measurements of all the temperature sensors 34 of the checking tool 10 so as to control that the temperature inside the checking chamber 12 is suitable (i.e. that in the checking chamber 12 an air bubble with relevant dimensions is not trapped, which would be indicative of an (possible/likely) unsatisfactory creation of the vacuum in the sterilization chamber 3).

Obviously, if the control unit 40 using the temperature sensors 34 of the checking tool 10 diagnoses an (possible/likely) unsatisfactory creation of the vacuum inside the sterilization chamber 3, it then interrupts the thermal treatment being carried out which has to be aborted (i.e. interrupted before its completion); in fact, if the control unit 40 using the temperature sensors 34 of the checking tool 10 diagnoses an (possible/likely) unsatisfactory creation of a vacuum in the sterilization chamber 3 there is no use in bringing to an end a thermal treatment which is already known to (potentially) be ineffective. In this situation, a new sterilization cycle is started from zero as soon as possible; depending on the type of load present in the sterilization chamber 3 it can be possible to restart a new sterilization cycle from the beginning without opening the sterilization chamber 3 (i.e. without any intervention of an operator) or it can be necessary to first ask for the intervention of an operator who opens the sterilization chamber 3 so as to recondition the load (for example replacing the sterilization bags previously used, and by now prejudiced, with new sterilization bags).

At the end of the sterilization amount of time (if the thermal treatment was ended in the absence of problems signalled by the checking tool 10), the control unit 40 interrupts the feeding of pressurized saturated steam into the sterilization chamber 3 and therefore the thermal treatment ends. It is thus necessary to wait for a certain amount of drying time during which the control unit 40 activates the vacuum generating device 7 which creates the vacuum inside the sterilization chamber 3 favouring the drying of the sterilized objects present in the sterilization chamber 3; it is also possible for the control unit 40 to activate electrical heaters (dry, i.e. without the use of steam) which by heating the sterilization chamber 3 favour the drying of the load present in the sterilization chamber 3.

At this point, while waiting for the sterilization chamber 3 to dry so as to be subsequently opened, the control unit 40 drives the thermoregulation system 27 so as to bring and maintain the checking chamber 11 to/at the incubation temperature (approximately 57°C for the *"Geobacillus Stearothermophilus").* The incubation temperature is maintained for a germination amount of time which could have a duration ranging between 10 and 30 minutes; i.e. the incubation temperature has to be sufficiently long so as to allow any survived *"Geobacillus Stearothermophilus"* spores to start germinating. To such regard, it is important to observe that in the liquid-state biological sterilization indicator 18 (besides a substance that changes its colour upon the varying of the pH) also a culture medium particularly suitable for the germination of the *"Geobacillus Stearothermophilus"* spores is present.

At the end of the germination amount of time (which is subsequent to the end of the sterilization amount of time), the control unit 40 activates the removing device 20 which sucks the quantity of liquid-state biological sterilization indicator 18 out of the test receptacle 17 and feeds the quantity of liquid-state biological sterilization indicator 18 into the analysis device 23. Then, the analysis device 23 analyses the quantity of liquid-state biological sterilization indicator 18 so as to check any presence of germination: if there is no presence of germination, it is established that the sterilization cycle took place correctly and it is thus possible to proceed with the opening of the sterilization chamber 3, otherwise if there is the presence of germination, it is established that the sterilization cycle did not take place correctly.

In this situation and as mentioned in the foregoing, a new sterilization cycle is started from zero and as soon as possible; depending on the type of load present in the sterilization chamber 3 it can be possible to restart a new sterilization cycle from the beginning without opening the sterilization chamber 3 (i.e. without any intervention of an operator) or it can be necessary to first ask for the intervention of an operator who opens the sterilization chamber 3 for reconditioning the load (for example replacing the sterilization bags previously used, and by now prejudiced, with new sterilization bags).

The embodiments described herein can be combined with one another without departing from the scope of protection of the present invention.

The above-described sterilization system 1 has numerous advantages.

First of all, the above-described sterilization system 1 allows checking in an automatic and completely objective manner (i.e. without any need of a manual intervention of an operator) the correct carrying out of a sterilization cycle. In this manner, the correct sterilization of the objects to be sterilized is certain and certifiable in an inalterable manner.

Furthermore, the above-described sterilization system 1 carries out all of its controls during the thermal treatment (by means of the checking tool 10) and immediately after the thermal treatment (by means of the checking tool 9) exploiting the *"dead time"* necessary for the drying and the cooling of the sterilization chamber 3; therefore, the above-described sterilization system 1 allows providing a definitive (objective and certified) evaluation of the success of the sterilization cycle already the moment in which the sterilization chamber 3 is opened for removing the objects just sterilized.

Finally, the above-described sterilization system 1 allows having a predetermined, certain and safe positioning of the test receptacle 17 or 37 containing the liquid-state biological sterilization indicator 18, totally eliminating the subjective component in the positioning of the test receptacle 17 or 37.

### LIST OF THE REFERENCE NUMERALS OF THE FIGURES

- 1: sterilization system
- 2: container
- 3: sterilization chamber
- 4: lower wall
- 5: upper wall
- 6: lateral wall
- 7: vacuum generating device
- 8: feeding device
- 9: checking tool
- 10: checking tool
- 11: checking chamber
- 12: checking chamber
- 13: through opening
- 14: vertical duct
- 15: vertical duct
- 16: forked duct
- 17: test receptacle
- 18: sterilization indicator
- 19: feeding device
- 20: removing device
- 21: inlet pipe
- 22: outlet pipe
- 23: analysis device
- 24: collecting device
- 25: feeding device
- 26: feeding device
- 27: thermoregulation system
- 28: cooling chamber
- 29: circulation device
- 30: inlet pipe
- 31: outlet pipe
- 32: electrical resistance
- 33: dead pipe
- 34: temperature sensor
- 35: housing
- 36: plug
- 37: test receptacle
- 38: protection container
- 39: plug
- 40: control unit

## Claims

1. An autoclave sterilization system (1) comprising:
a container (2), which encloses a sterilization chamber (3) and has at least one wall (6) delimiting the sterilization chamber (3);
a vacuum generating device (7), which is configured to create a vacuum inside the sterilization chamber (3);
a first feeding device (8) to feed saturated steam into the sterilization chamber (3); and
at least one tool (9, 10) to check the sterilization process;
the sterilization system (1) is **characterized in that:**
the wall (6) comprises a through opening (13) directly communicating with the sterilization chamber (3);
at least one checking chamber (11, 12) is provided, which is arranged on the outside of the sterilization chamber (3) and is configured to at least partially house the checking tool (9, 10); and
a vertical duct (14, 15) is provided, which is connected to the through opening (13) and ends, at the opposite end relative to the through opening (13), in the checking chamber (11, 12) so as to lead, during a sterilization treatment, the saturated steam present in the sterilization chamber (3) into the checking chamber (11, 12).

2. The sterilization system (1) according to claim 1, wherein the checking chamber (11, 12) is arranged at a vertical height that is greater than a vertical height of a ceiling of the sterilization chamber (3).

3. The sterilization system (1) according to claim 1 or 2, wherein:
the checking tool (9) comprises a test receptacle (17), which is configured to contain a liquid-state biological sterilization indicator (18), is arranged in the checking chamber (11) and is pneumatically and hydraulically isolated relative to the checking chamber (11); and
the checking chamber (11) directly communicates with the vertical duct (14) so that, during a sterilization treatment, the checking chamber (11) is filled with the saturated steam present in the sterilization chamber (3), which brushes the test receptacle (17) from the outside.

4. The sterilization system (1) according to claim 3, wherein:
the test receptacle (37) is disposable; and
the checking chamber (11, 12) is configured to house the test receptacle (37) in a removable manner and has an openable lid, which can be opened to insert or remove a test receptacle (37).

5. The sterilization system (1) according to claim 3, wherein:
the test receptacle (17) is reusable;
the checking chamber (11) is configured to house the test receptacle (17) in a permanent manner, namely in a non-removable manner;
the checking tool (9) comprises a second feeding device (19), which is configured to feed, before carrying out a sterilization treatment, a quantity of liquid-state biological sterilization indicator (18) into the test receptacle (17); and
the checking tool (9) comprises a removing device (20), which is configured to suck, after carrying out a sterilization treatment, the quantity of liquid-state biological sterilization indicator (18) out of the test receptacle (17).

6. The sterilization system (1) according to claim 5, wherein the checking tool (9) comprises an analysis device (23), which is configured to receive the quantity of liquid-state biological sterilization indicator (18) from the removing device (20) and to analyse the liquid-state biological sterilization indicator (18) so as to check whether a sterilization treatment had a positive result.

7. The sterilization system (1) according to claim 5 o 6, wherein the checking tool (9) comprises:
a third feeding device (25), which is configured to feed a washing liquid into the test receptacle (17); and/or
a fourth feeding device (26), which is configured to feed a sterilization liquid into the test receptacle (17).

8. The sterilization system (1) according to one of the claims from 3 to 7, wherein the checking tool (9) comprises a thermoregulation system (27), which is configured to keep the checking chamber (11) containing the test receptacle (17) at a desired incubation temperature.

9. The sterilization system (1) according to claim 8, wherein the thermoregulation system (27) comprises a liquid cooling device provided with:
a cooling chamber (28), which surrounds the checking chamber (11) and is isolated from the checking chamber (11); and
a circulation device (29), which is configured to have a cooling liquid circulate in the cooling chamber (28).

10. The sterilization system (1) according to claim 8 o 9, wherein the thermoregulation system (27) comprises at least one electrical resistance (32), which is configured to heat the checking chamber (11).

11. The sterilization system (1) according to claim 1 or 2, wherein:
the checking chamber (12) is defined in a dead pipe (33), which is arranged vertically so as to place at the top a closed end and so as to place at the bottom an open end, which directly communicates with the vertical duct (15), so that, during a sterilization treatment, the saturated steam fills the checking chamber (12); and
the checking tool (10) comprises at least one temperature sensor (34), which is coupled to the dead pipe (33) so as to detect a temperature inside the checking chamber (12) and close to the closed end of the dead pipe (33) .

12. The sterilization system (1) according to claim 11, wherein the checking tool (10) comprises a plurality of, in particular four, temperature sensors (34), which are arranged at different heights of the dead pipe (33) so as to detect respective temperatures inside the checking chamber (12) and close to the closed end of the dead pipe (33).

13. The sterilization system (1) according to claim 11 or 12, wherein the checking chamber (12) has, between the temperature sensor (34) and its closed end, a volume ranging between 1.5 and 4.5 cm³.

14. The sterilization system (1) according to one of the claims from 1 to 13 and comprising:
two different checking tools (9, 10);
two different vertical ducts (14, 15), which are adjacent and parallel to one another and both preferably originate from the same through opening (13) by means of a forked duct (16); and
two checking chambers (11, 12), each connected to a corresponding vertical duct (14, 15) and configured to at least partially house a respective checking tool (9, 10).

15. A method to carry out a sterilization cycle by means of an autoclave sterilization system (1) according to claim 1; wherein the method comprises the steps of:
inserting at least one object to be sterilized into the sterilization chamber (3);
automatically feeding a quantity of liquid-state biological sterilization indicator (18) into a test receptacle (17) of the checking tool (9) arranged in the checking chamber (11);
creating a vacuum in the sterilization chamber (3) and, as a consequence, also in the checking chamber (11);
feeding pressurized saturated steam into the sterilization chamber (3) and, as a consequence, also into the checking chamber (11, 12) and maintaining the presence of the pressurized saturated steam for a predetermined sterilization amount of time;
interrupting, at the end of the sterilization amount of time, the feeding of the pressurized saturated steam into the sterilization chamber (3);
automatically extracting, at the end of the sterilization amount of time, the quantity of liquid-state biological sterilization indicator (18) from the test receptacle (17); and
analysing, by means of an analysis device (23) arranged on the outside of the checking chamber (11), the quantity of liquid-state biological sterilization indicator (18) in order to determine whether there is a germination activity so as to establish whether the sterilization cycle correctly took place.

16. The method according to claim 15 and comprising the further steps of:
cooling, at the end of the sterilization amount of time and before extracting the quantity of liquid-state biological sterilization indicator (18), the checking chamber (11) up to an incubation temperature;
keeping the checking chamber (11) at the incubation temperature for a predetermined germination amount of time; and
automatically extracting the quantity of liquid-state biological sterilization indicator (18) from the test receptacle (17) only at the end of the germination amount of time.
